# EUROPÄISCHE PATENTSCHRIFT

(11) **EP 3 268 742 B1**
(45) Veröffentlichungstag und Bekanntmachung des Hinweises auf die Patenterteilung: **01.05.2019**
(21) Anmeldenummer: 16738349.6
(22) Anmeldetag: 11.03.2016
(51) Int. Cl.: G01N 33/569

(54) **VERFAHREN ZUM VERBESSERTEN NACHWEIS ANTIGEN-SPEZIFISCHER, GEWEBEINFILTRIERENDER IMMUNZELLEN**
METHOD FOR THE IMPROVED DETECTION OF ANTIGEN-SPECIFIC, TISSUE-INFILTRATING IMMUNE CELLS
PROCÉDÉ POUR L'IDENTIFICATION AMÉLIORÉE DE CELLULES IMMUNITAIRES SPÉCIFIQUES D'ANTIGÈNES, INFILTRANT LES TISSUS

(30) Priorität: 12.03.2015 DE 102015003141
(43) Veröffentlichungstag der Anmeldung: 17.01.2018
(73) Patentinhaber: Genome Identification Diagnostics GmbH, 72479 Strassberg (DE)
(72) Erfinder: SESTER, Martina, 66424 Homburg/Saar (DE)
(74) Vertreter: Patentanwälte Ruff, Wilhelm, Beier, Dauster & Partner mbB
(86) Internationale Anmeldenummer: PCT/DE2016/000111
(87) Internationale Veröffentlichungsnummer: WO 2016/173573

(56) Entgegenhaltungen:
- RACHEL PERRET ET AL: "Analysis of Tumor-infiltrating Lymphocytes Following CD45 Enrichment", BIO-PROTOCOL, Bd. 4, Nr. 16, 20. August 2014 (2014-08-20), Seiten 1-9, XP055293148,
- Motawa E El-Houseini ET AL: "Isolation and Immuno-Phenotypic Characterization of Tumor Infiltrating Lymphocytes (TILs) Obtained from Breast Malignant Tumor Tissues of Egyptian Patients", Journal of the Egyptian National Cancer Institute, 1. Dezember 2008 (2008-12-01), Seiten 379-386, XP055293191, Egypt Gefunden im Internet: URL:http://www.nci.cu.edu.eg/Journal/Dec20 08/Can_9.pdf [gefunden am 2016-08-03]
- SCHMIDT TINA ET AL: "Detection of antigen-specific T cells based on intracellular cytokine staining using flow-cytometry.", METHODS IN MOLECULAR BIOLOGY (CLIFTON, N.J.) 2013, Bd. 1064, 2013, Seiten 267-274, XP009191236, ISSN: 1940-6029 in der Anmeldung erwähnt

## Beschreibung

Infektionen, Tumorerkrankungen, Abstoßungsreaktionen oder Autoimmunerkrankungen sind mit Infiltrationen von Antigen-spezifischen T Zellen in Gewebe assoziiert, deren Nachweis prognostische Bedeutung für die Beurteilung der jeweiligen Erkrankung hat. Der Nachweis der T Zellen erfolgt bislang histologisch im Gewebeschnitt oder nach Isolation von Zellen aus Gewebe. Diese Techniken sind jedoch nicht oder nur begrenzt in der Lage, die Antigen-Spezifität der im Gewebe befindlichen T Zellen zu bestimmen. Übliche Stimulationsverfahren zum Nachweis Antigen-spezifischer T Zellen, wie sie im Blut durchgeführt werden, sind nicht mit isolierten, geringen Mengen an T Zellen aus Gewebe möglich (1).

Basierend auf einem kürzlich entwickelten Verfahren zum verbesserten Nachweis von Antigen-spezifischen T Zellen aus extrasanguinen Flüssigkeiten durch kombinierte Stimulation mit Vollblut (s. Patentanmeldung aus unserem Labor mit dem Titel "Neues Verfahren zum verbesserten Nachweis Antigen-spezifischer Immunzellen in extrasanguinen Flüssigkeiten" beim Deutschen Patentamt, veröffentlicht am 30.7.2015 als Patentschrift DE 10 2014 018 047 B3) ist davon auszugehen, dass sich der Nachweis Antigen-spezifischer T Zellen aus Gewebe auf ähnliche Weise durch kombinierte Stimulation der aus Gewebe isolierten Zellen mit Vollblut derselben Person optimieren lässt. Die Quantifizierung der Antigen-spezifischen Zellen erfolgt dabei mittels Durchflusszytometrie anhand der spezifischen Zytokininduktion von T-Lymphozyten nach Stimulation (2, 3). Zusätzlich bietet die durchflusszytometrische Multiparameteranalyse die Möglichkeit zur Charakterisierung der Funktionalität und Differenzierung der gewebeinfiltrierenden Immunzellen durch Anfärben einer Vielzahl von Zytokinen und Oberflächenmolekülen.

Bislang geläufige Verfahren zur Antigen-spezifischen Stimulation isolierter T Zellen erfordern eine zusätzliche aufwändige Isolation von Antigen-präsentierenden Zellen, die den T Zellen zur Stimulation zugegeben werden. In der hier vorliegenden Erfindung werden ähnlich wie bei extrasanguinen Flüssigkeiten (s. Patentschrift DE 10 2014 018 047 B3) die aus Gewebe isolierten Zellen und Vollblut-Proben derselben Person vermischt und anschließend simultan mit dem interessierenden Antigen stimuliert. Dies führt dazu, dass die im Blut befindlichen Antigen-präsentierenden Zellen sowohl Antigen-spezifische T-Zellen aus dem Blut als auch aus dem Gewebe aktivieren und eine spezifische Zytokininduktion hervorrufen, anhand derer die Antigen-spezifischen T-Zellen identifiziert werden können. Das besondere Merkmal des neuen Analyseverfahrens ist dabei, dass die aus Gewebe isolierten Zellen mit einem Fluorochrom-gekoppelten CD45-Antikörper angefärbt werden, bevor sie mit den Blutzellen vermengt werden. Dies führt dazu, dass bei der abschließenden Analyse T-Zellen aus dem Blut und T-Zellen aus dem Gewebe aufgrund des Fehlens bzw. des Vorhandenseins dieses Fluoreszenzsignals diskriminiert werden können, obwohl die Zellen während der Verarbeitung miteinander vermischt waren. Durch die simultane Stimulation ergibt sich ein verbesserter Nachweis eines Signals, das durch alleinige Stimulation der Gewebe T Zellen nicht oder schlechter detektierbar ist. Das Vollblut liefert folglich das für die Stimulation und Induktion einer T Zellantwort notwendige physiologische Milieu, das bei alleiniger Stimulation der isolierten Gewebezellen nicht optimal ist. In 4 und 5 werden ebenfalls Verfahren zum Nachweis von aus Geweben isolierten Antigen-spezifischen Immunzellen offenbart.

Durch die Erfindung wird das technische Problem gelöst, dass gewebeinfiltrierende T Zellen bislang zwar identifiziert werden können, der Nachweis ihrer Antigen-Spezifität jedoch insbesondere bei geringen Zellzahlen nicht möglich oder erschwert ist. Das neue Testverfahren ermöglicht also eine sensitivere quantitative und qualitative Analyse Antigen-spezifischer T-Zellen aus Geweben, die als diagnostisches Nachweisverfahren und zum Monitoring des Behandlungserfolgs Erreger-spezifischer, aber auch durch gegen Tumorantigene, Autoantigene oder Alloantigene gerichtete T-Zellen bedingte Organ-Erkrankungen Anwendung finden könnte. Zudem erlaubt diese technische Erfindung die Möglichkeit zur simultanen Quantifizierung und Analyse Antigen-spezifischer T-Zellen aus Gewebe und Blut und bietet somit die Möglichkeit, in einer einzigen Stimulationsreaktion Verschiebungen Antigen-spezifischer T-Zellen im Gewebe im Vergleich zum Blut zu ermitteln. Dieses Testverfahren kann daher einen entscheidenden Beitrag zur Diagnostik bei Patienten mit Infektionen, Autoimmunerkrankungen, nach Organtransplantation oder bei Tumoren leisten.

### Schlagwörter

zelluläre Immunität, virale Infektion, gewebeinfiltrierende Immunzellen, tumorinfiltrierende Lymphozyten, Vollblut, Immundiagnostik, Autoimmunerkrankung, Tumorerkrankung

### Zitierte Nichtpatentliteratur

1. Protti, M.P., Monte, L.D., and Lullo, G.D. 2014. Tumor antigen-specific CD4+ T cells in cancer immunity: from antigen identification to tumor prognosis and development of therapeutic strategies. Tissue Antigens 83:237-246.
2. Schmidt, T., and Sester, M. 2013. Detection of antigen-specific T cells based on intracellular cytokine staining using flow-cytometry. Methods Mol Biol 1064:267-274.
3. Letsch, A., and Scheibenbogen, C. 2003. Quantification and characterization of specific T-cells by antigen-specific cytokine production using ELISPOT assay or intracellular cytokine staining. Methods 31:143-149.
4. Perret, R. et al. 2014. Analysis of Tumor-infiltrating Lymphocytes Following CD45 Enrichment. Bio-Protocol 4(16):1-9.
5. El-Houseini et al. 2008. Isolation and Immuno-Phenotypic Characterization of Tumor Infiltrating Lymphocytes (TILs) Obtained from Breast Malignant Tumor Tissues of Egyptian Patients. Journal of the Egyptian National Cancer Institute 20(4):379-386.

## Patentansprüche

1. Verfahren zum verbesserten Nachweis von aus Geweben isolierten Antigen-spezifischen Immunzellen, bestehend aus folgenden Arbeitsschritten:
- Isolierung der vitalen Immunzellen aus Gewebe durch geeignete Präparationstechniken, beispielsweise durch enzymatischen oder mechanischen Aufschluss,
- Bestimmung der Gesamtzellzahl, beispielsweise werden je Stimulationsansatz 10⁴ bis 1,5x10⁶ Zellen benötigt,
- Vorfärbung der isolierten Immunzellen mit einem Fluorochrom-gekoppelten Antikörper, welcher gegen auf den Zellen exprimierte Antigene, z.B. CD45, gerichtet ist oder mittels für die Färbung von vitalen Zellen geeigneten Fluorochromen, z.B. Carboxyfluorescein Diacetat Succinimidyl Ester, CFDA-SE,
- Entfernung ungebundener Antikörper oder Fluorochrome durch Waschschritte,
- Vermischen der vorgefärbten Immunzellen mit heparinisiertem Vollblut derselben Person,
- Stimulierung der gemischten Proben mit dem interessierenden Antigen, mit oder ohne Zusatz von ko-stimulatorischen Antikörpern anti-CD49d und anti-CD28, anti-CD49d alleine oder anti-CD28 alleine,
- Inkubation für 30min bis 4h bei 35 bis 39°C und 3 bis 10% CO₂,
- Zugabe eines Sekretionsinhibitors, z.B. Brefeldin A, Monensin oder andere,
- Inkubation für 2 bis 12 Stunden bei 35 bis 39°C und 3 bis 10% CO₂,
- Lyse von Erythrozyten und Fixierung der Leukozyten,
- Waschschritte zur Isolierung der Leukozyten,
- Erhöhung der Zellmembrandurchlässigkeit durch Zugabe einer Zellmembran-Permeabilität-erhöhenden Substanz, z.B. Saponin,
- Inkubation mit spezifischen Fluorochrom-markierten Antikörpern gegen Aktivierungsmarker, z.B. anti-CD69, anti-IFN-γ, für mindestens 10min auf Eis oder bei Raumtemperatur oder bei 35 bis 39°C,
- Erneute Waschschritte zur Entfernung ungebundener Antikörper,
- Analyse der Zellen an einem Durchflusszytometer und Diskriminierung der Immunzellen aus dem Gewebe von denen aus dem Blut anhand des Fluoreszenzsignals, das zur Vorfärbung verwendet wurde, z.B. Fluorochrom des CD45-Antikörpers, CFSE, wobei die gegen den Stimulus reagierenden Zellen anhand der Positivität in Bezug auf die Aktivierungsmarker von den nicht reagierenden Zellen unterschieden werden.

2. Verfahren gemäß Patentanspruch 1, wobei ein Ansatz als Negativkontrolle durchgeführt wird, der bis auf die Zugabe von Antigen identisch ist zu besagtem Ansatz aus Patentanspruch 1 und bei dem statt des Antigens die Zugabe einer entsprechenden Menge eines physiologischen Puffers bzw. Lösungsmittels, z.B. PBS, oder eines nicht-infizierten Zell-Lysates bzw. eines nicht immunstimulierenden Protein- oder Peptidgemisches zu den Immunzellen aus Gewebe und Blut erfolgt.

3. Verfahren gemäß Patentanspruch 2, wobei eine Positivkontrolle durchgeführt wird durch Zugabe eines polyklonalen Stimulus, z.B. *Staphylococcus aureus* Enterotoxin B, SEB, zu den Immunzellen aus Gewebe und Blut in einem dritten Ansatz, der ansonsten identisch ist zu besagtem ersten Ansatz.

4. Verfahren gemäß zumindest einem der Patentansprüche 1 bis 3, wobei das Verhältnis aus dem relativen Anteil der durch das Antigen aktivierten Immunzellen im Gewebe zu dem relativen Anteil der durch das Antigen aktivierten Immunzellen im Blut ein Maß für die Immunaktivität in dem Gewebe darstellt, aus dem die Immunzellen gewonnen wurden.

5. Verfahren gemäß zumindest einem der Patentansprüche 1 bis 4, wobei Gewebe und Blut menschlichen Ursprungs sind.

6. Verfahren gemäß zumindest einem der Patentansprüche 1 bis 5, wobei die vorgefärbten Immunzellen mit 100 µl bis 1500 µl heparinisiertem Vollblut je Stimulationsansatz vermischt werden.

## Claims

1. Method for improved detection of antigen-specific immune cells isolated from tissues, comprising the following procedure steps:
- isolating of vital immune cells from tissue using appropriate preparation techniques, for example, using enzymatic or mechanical extraction,
- determining the total cell count, for example 10⁴ to 1.5x10⁶ cells are needed per stimulation batch,
- prestaining of the isolated immune cells using a fluorochrome-coupled antibody which is aimed at the antigens expressed on the cells, e.g. CD45, or using fluorochromes capable of staining vital cells, e.g. carboxyfluorescein diacetate succinimidyl ester, CFDA-SE,
- removal of unbound antibodies or fluorochromes by washing steps,
- mixing of the prestained immune cells with heparinized whole blood of the same individual patient,
- stimulating the mixed samples with the interesting antigen, with or without addition of co-stimulatory antibodies anti-CD49d and anti-CD28, anti-CD49d alone or anti-CD28 alone,
- incubation for 30min to 4h at 35 to 39°C and 3 to 10% CO₂,
- adding of a secretion inhibitor, e.g. brefeldin A, monensin or others,
- incubation for 2 to 12 hours at 35 to 39°C and 3 to 10% CO₂,
- lysis of erythrocytes and fixation of the leucocytes,
- washing steps for isolating the leucocytes,
- increasing cell membrane permeability by addition of a cell membrane permeability increasing substance, e.g. saponin,
- incubation with specific fluorochrome-marked antibodies against activation markers, e.g. anti-CD69, anti-IFN-y, for at least 10min on ice or at room temperature or at 35 to 39°C,
- repeated washing steps for removing unbound antibodies,
- analysis of cells on a flow cytometry device and discriminating the immune cells from tissue against those from blood based on the fluorescence signal which was used for the prestaining, e.g. fluorochrome of the CD45 antibody, CFSE, wherein the cells responding to the stimulus are distinguished from the non-responding cells based on the positivity in relation to the activation markers.

2. Method according to claim 1, wherein one batch is performed as a negative control, which is identical to said batch of claim 1 with the exception of the addition of antigen, and wherein instead of the antigen the addition of a corresponding amount of a physiological buffer or solvent, respectively, e.g. PBS, or a not infected cell lysate or a not immune stimulating protein or peptide mixture to the immune cells from tissue and blood is performed.

3. Method according to claim 2, wherein a positive control is performed by addition of a polyclonal stimulus, e.g. *Staphylococcus aureaus* enterotoxin B, SEB, to the immune cells from tissue and blood in a third batch, which otherwise is identical to said first batch.

4. Method according to at least one of claims 1 to 3, wherein the ratio of the relative proportion of the immune cells activated by the antigen in tissue to the relative proportion of the immune cells activated by the antigen in blood is a measure for the immune activity in the tissue from which the immune cells were taken.

5. Method according to at least one of claims 1 to 4, wherein tissue and blood are of human origin.

6. Method according to at least one of claims 1 to 5, wherein the prestained immune cells are mixed with 100 µl to 1500 µl of heparinized whole blood per stimulation batch.

## Revendications

1. Procédé de détection améliorée de cellules immunitaires spécifiques à des antigènes isolées à partir de tissus, constitué par les étapes suivantes :
- l'isolement des cellules immunitaires vivantes à partir d'un tissu par des techniques de préparation appropriées, par exemple par désintégration enzymatique ou mécanique,
- la détermination du nombre total de cellules, 10⁴ à 1,5 x 10⁶ cellules étant par exemple nécessaires par préparation de stimulation,
- la pré-coloration des cellules immunitaires isolées avec un anticorps couplé à un fluorochrome, qui est dirigé contre des antigènes exprimés sur les cellules, p. ex. CD45, ou au moyen de fluorochromes appropriés pour la coloration de cellules vivantes, p. ex. l'ester de succinimidyle de diacétate de carboxyfluorescéine, CFDA-SE,
- l'élimination des anticorps ou fluorochromes non reliés par des étapes de lavage,
- le mélange des cellules immunitaires pré-colorées avec du sang entier hépariné de la même personne,
- la stimulation de l'échantillon mélangé avec l'antigène d'intérêt, avec ou sans ajout d'anticorps co-stimulants anti-CD49d et anti-CD28, anti-CD49d seuls ou anti-CD28 seuls,
- l'incubation pendant 30 minutes à 4 heures à 35 à 39 °C et 3 à 10 % de CO₂,
- l'ajout d'un inhibiteur de sécrétion, p. ex. la bréfeldine A, la monensine ou un autre,
- l'incubation pendant 2 à 12 heures à 35 à 39 °C et 3 à 10 % de CO₂,
- la lyse d'érythrocytes et la fixation des leucocytes,
- des étapes de lavage pour l'isolement des leucocytes,
- l'augmentation de la perméabilité de la membrane cellulaire par ajout d'une substance augmentant la perméabilité de la membrane cellulaire, p. ex. la saponine,
- l'incubation avec des anticorps marqués par des fluorochromes spécifiques contre un marqueur d'activation, p. ex. anti-CD69, anti-IFN-y, pendant au moins 10 minutes sur de la glace ou à température ambiante ou à 35 à 39 °C,
- de nouvelles étapes de lavage pour l'élimination d'anticorps non reliés,
- l'analyse des cellules avec un cytomètre en flux et la discrimination des cellules immunitaires du tissu de celles du sang à partir du signal de fluorescence qui a été utilisé pour la pré-coloration, p. ex. le fluorochrome de l'anticorps CD45, CFSE, les cellules réagissant au stimulus étant différenciées des cellules ne réagissant pas à partir de la positivité au regard du marqueur d'activation.

2. Procédé selon la revendication 1, dans lequel une préparation est réalisée en tant que témoin négatif, qui est identique à ladite préparation de la revendication 1 à l'exception de l'ajout d'un antigène et pour laquelle, au lieu de l'antigène, l'ajout d'une quantité correspondante d'un tampon physiologique ou d'un solvant, p. ex. le PBS, ou d'un lysat cellulaire non infiltré ou d'un mélange de protéines ou de peptides non-immunostimulant, aux cellules immunitaires issues du tissu et du sang a lieu.

3. Procédé selon la revendication 2, dans lequel un témoin positif est réalisé par ajout d'un stimulus polyclonal, p. ex. l'entérotoxine B de *Staphylococcus aureus,* SEB, aux cellules immunitaires issues du tissu et du sang dans une troisième préparation, qui est sinon identique à ladite première préparation.

4. Procédé selon au moins l'une quelconque des revendications 1 à 3, dans lequel le rapport entre la proportion relative des cellules immunitaires activées par l'antigène dans le tissu et la proportion relative des cellules immunitaires activées par l'antigène dans le sang est une mesure de l'activité immunitaire dans le tissu à partir duquel les cellules immunitaires ont été obtenues.

5. Procédé selon au moins l'une quelconque des revendications 1 à 4, dans lequel le tissu et le sang sont d'origine humaine.

6. Procédé selon au moins l'une quelconque des revendications 1 à 5, dans lequel les cellules immunitaires pré-colorées sont mélangées avec 100 µl à 1 500 µl de sang entier hépariné par préparation de stimulation.
